# EUROPEAN PATENT SPECIFICATION

(11) **EP 1 437 336 B1**
(45) Date of publication and mention of the grant of the patent: **30.05.2012**
(21) Application number: 01972566.2
(22) Date of filing: 28.09.2001
(51) Int. Cl.: C07C 7/08, C07C 7/20, C07C 11/167, C07C 11/18

(54) **METHOD OF SEPARATING/PURIFYING CONJUGATED DIENE AND SEPARATION/PURIFICATION APPARATUS**
VERFAHREN ZUR TRENNUNG/REINIGUNG KONJUGIERTER DIENE UND TRENNUNGS-/REINIGUNGSAPPARAT
PROCEDE DE SEPARATION/PURIFICATION DE DIENE CONJUGUE ET APPAREIL DE SEPARATION/PURIFICATION

(43) Date of publication of application: 14.07.2004
(73) Proprietor: Zeon Corporation, Tokyo 100-8246 (JP)
(72) Inventor: KANAUCHI, Masanobu, Tokyo 100-8323 (JP); KAJI, Masayoshi, Kurashiki-shi, Okayama 711-0934 (JP)
(74) Representative: Vossius & Partner
(86) International application number: PCT/JP2001/008516
(87) International publication number: WO 2003/031377

(56) References cited:
- EP-A1- 1 083 160
- JP-A- 10 251 662
- JP-A- 58 032 685
- DATABASE WPI Week 197710 Thomson Scientific, London, GB; AN 1977-17643Y XP002549071 & NL 152 237 B (JAPANESE GEON CO LTD) 15 February 1977 (1977-02-15)

## Description

### TECHNICAL FIELD

The present invention relates to a method and apparatus for separating and purifying conjugated dienes capable of efficiently suppressing generation of rubbery substances inside a purification apparatus when separating and purifying high purity isoprene, butadiene and other conjugated dienes from petroleum distillates.

### BACKGOUND ART

Conjugated dienes, such as 1,3-butadiene, isoprene and chloroprene as unsaturated hydrocarbons, are liable to be homopolymerized or copolymerized with other unsaturated compounds which can be copolymerized in steps of distillation, extractive distillation, extraction, hydrogenation processing, hydrogenation purification and heat treatment, etc. and when being stored, carried and processed, etc.

Conjugated diene is included in a hydrocarbon mixture of C2 or more after removing methane, hydrogen and nitrogen, etc. from a cracked gas and an off-gas exhausted from a pyrolysis furnace; a hydrocarbon mixture of C3 or more after separating and purifying ethylene by a cryogenic distillation method, etc.; a C4 hydrocarbon fraction; a C5 hydrocarbon fraction; and a hydrocarbon mixture of C6 or more after removing a C4 hydrocarbon fraction and a C5 hydrocarbon fraction; etc.

In an apparatus for separating and purifying these hydrocarbon fractions and hydrocarbon mixtures, rubbery substances considered to be generated by polymerization or copolymerization of conjugated diene are adhered inside the apparatus to cause blockage, particularly, they are liable to be generated in an evaporator and at a bottom of a distillation column where the hydrocarbon mixture is exposed to a high temperature, thus, the apparatus has to be stopped on regular or irregular basis to get rid of the blockage by cleaning the inside.

Therefore, there are demands for a method of preventing polymerization or copolymerization of conjugated dienes inside a separation and purification apparatus for a hydrocarbon mixture including conjugated diene to suppress blockages.

As a method of preventing polymerization of conjugated diene inside a separation and purification apparatus, a variety of proposals have been made.

For example, the Japanese Unexamined Patent Publication No. 50-112304 discloses a method of distilling C5 hydrocarbon in the presence of a di-lower alkylhydroxylamine.

Also, the Japanese Unexamined Patent Publication No. 56-81526 and the Japanese Examined Patent Publication No. 43-20281 disclose a method of extracting and distilling in the presence of furfural and a furfural condensation product in an extracting solvent.

Furthermore, the Japanese Examined Patent Publication No.47-41323 and No.45-19682 disclose a method of extracting and distilling conjugated diene hydrocarbon by adding a polymerization inhibitor or a polymerization chain transfer agent in an extracting solvent.

Furthermore, the Japanese Examined Patent Publication No. 49-6886 and No. 49-7126 disclose a method of preventing polymerization of conjugated diene hydrocarbon by adding an organic phosphoric ester compound.

However, according to the method described in the publication, a more than necessary amount of a polymerization inhibitor is added to suppress generation of rubbery substances inside the apparatus in some cases, consequently, costs of separation and purification tend to rise.

JP 10-251662 A discloses a method for separating conjugated dienes by extractive distillation of a petroleum fraction containing conjugated dienes with an extractive distillation tower, the oxygen concentration in the fraction and in an extractant are measured; the weight of oxygen inflow is calculated from the measured oxygen concentrations and the amounts of the fraction and extractant supplied; and the amount of a polymerization inhibitor supplied to the tower is controlled in proportion to the oxygen inflow weight.

### DISCLOSURE OF THE INVENTION

An object of the present invention is to provide a method and apparatus for separating and purifying conjugated dienes, capable of efficiently and stably suppressing generation of rubbery substances in the apparatus, by which an excessive polymerization inhibitor is not left in the conjugated dienes.
(1) To attain the above object, according to a first aspect, a method of separating and purifying conjugated dienes, includes
   a step of obtaining a first bottom product by taking out a hydrocarbon mixture including conjugated diene under an environment in the presence of a polymerization inhibitor including at least one of nitrite and di-lower alkylhydroxylamine as defined below by chemical formula 1 in an extractive distillation column;
   a step of obtaining a second bottom product by distilling the first bottom product in a diffusion column;
   a step of recovering the second bottom product and supplying to the extractive distillation column;
   a step of measuring concentration of a polymerization inhibitor included in the second bottom product; and
   a control step of controlling the concentration of the polymerization inhibitor included in the second bottom product by changing a supply amount of the polymerization inhibitor to the extractive distillation column in accordance with the measured concentration of the polymerization inhibitor.
(2) To attain the above object, an apparatus for separating and purifying conjugated dienes according to the first aspect comprises
   an extractive distillation column for obtaining a first bottom product by taking out a hydrocarbon mixture including conjugated diene under an environment in the presence of a polymerization inhibitor including at least one of nitrite and di-lower alkylhydroxylamine as defined by chemical formula 1 below;
   a diffusion column for obtaining a second bottom product by distilling the first bottom product;
   a first supply system for recovering the second bottom product and supplying to the extractive distillation column;
   a measurement means for measuring concentration of a polymerization inhibitor included in the second bottom product;
   a second supply system for supplying the polymerization inhibitor newly to the extractive distillation column; and
   a control means for controlling the concentration of the polymerization inhibitor included in the second bottom product by changing a supply amount of the polymerization inhibitor to the extractive distillation column in accordance with the measured concentration of the polymerization inhibitor.
(3) In the present invention, it is preferable to use an analyzing apparatus, such as an ion chromatograph, liquid chromatograph, and gas chromatograph, for measuring concentration of the polymerization inhibitor, and more preferably, a liquid chromatograph analyzing apparatus is used.
   In the present invention, as a method of changing a supply amount of a polymerization inhibitor to an extractive distillation column, for example, when the measured concentration of the polymerization inhibitor exceeds the upper limit value of a reference range, the supply amount of the polymerization inhibitor to the extractive distillation column is decreased, while when the measured concentration of the polymerization inhibitor is below the lower limit value of the reference range, the supply amount of the polymerization inhibitor to the extractive distillation column is increased.
   In the present invention, a means to change the supply amount of the polymerization inhibitor to the extractive distillation column may be manually controlled or automatically controlled.
(4) A hydrocarbon mixture including conjugated diene, which can be applied to the present invention is not particularly limited, but is normally a hydrocarbon mixture of C4 or more obtained by cracking naphtha and separating ethylene, propylene and other C2 and C3 hydrocarbons, preferably a C4 hydrocarbon fraction or C5 hydrocarbon fraction, more preferably a C4 hydrocarbon fraction including butadiene or a C5 hydrocarbon fraction including isoprene.
(5) A polymerization inhibitor which can be used in the present invention is used for suppressing generation of rubbery substances inside the apparatus and may contain (a) nitrite and/or (b) di-lower alkylhydroxylamine as defined in formula 1 below, furthermore, (c) a phosphorous-containing compound as defined below.
   (a) As nitrite, sodium nitrite and potassium nitrite, etc. may be mentioned.
      As a method of supplying nitrite, in terms of operationality and supply speed, it is preferably dissolved in water or a polar solvent, such as dimethylformamide (DMF), to be supplied, and in terms of dissolubility, it is particularly preferable to be dissolved in water to be supplied.
   (b) The di-lower alkylhydroxylamine, is expressed by the chemical formula 1 below or example compounds such as diethylhydroxylamine, dimethylhydroxylamine, methylethylhydroxylamine, dipropylhydroxylamine, dibutylhydroxylamine and dipentylhydroxylamine, may be mentioned. 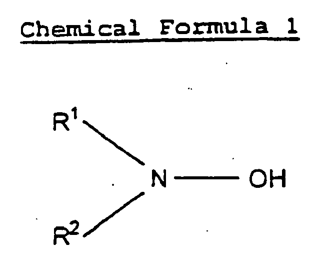 (Note that R¹ and R² in the chemical formula 1 are linear, branched or cyclic alkyl groups having 1 to 10 carbon atoms, preferably 1 to 6 carbon atoms, respectively. As an alkyl group, a methyl group, an ethyl group, a propyl group, an isopropyl group, a butyl group, a pentyl group and a cyclohexyl group, etc. may be mentioned.)
   (c) The phosphorous-containing compound is selected from phosphoric acid, phosphonic acid, phosphinic acid, diphosphonic acid, diphosphoric acid, tripolyphosphoric acid and metaphosphoric acid; alkyl dihydrogenphosphate described by the chemical formula 2, dialkyl hydrogenphosphate expressed by the chemical formula 3, and trialkyl phosphate expressed by the chemical formula 4, 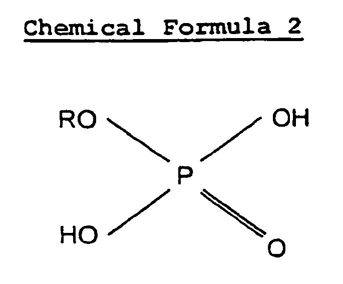 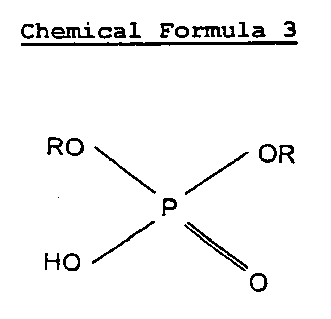 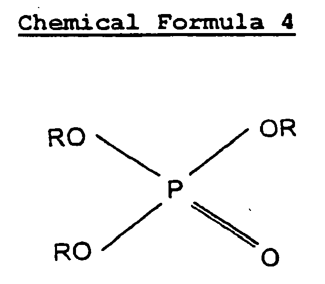 (Note that R in the chemical formulas 2 to 4 includes hydrocarbon groups such as an alkyl group, a phenyl group and an alkylphenyl; and hydrophobic groups such as an alkylphenyl group, a polyethylene oxide group and an alkylphenyl polyethylene oxide group), triphenylphosphate expressed by the chemical formula 5, tris(nonylphenyl)phosphite expressed by the chemical formula 6, dimethyl phosphonate, diethyl phosphonate, triisopropyl phosphonate, triphenyl phosphonate, metaphosphate, phosphoric ester based surfactants expressed by the chemical formulas 7, 8, 9 and 10, and a compound mixture expressed by the chemical formula 11 and other esterification products of phosphoric compounds; 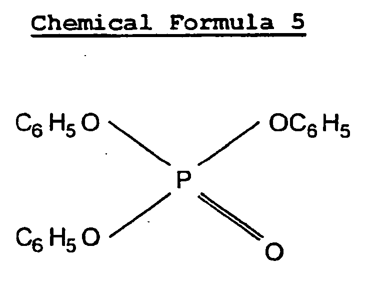 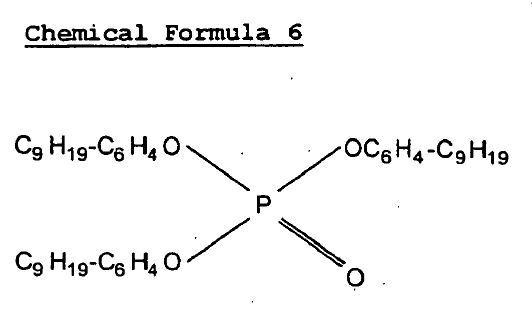 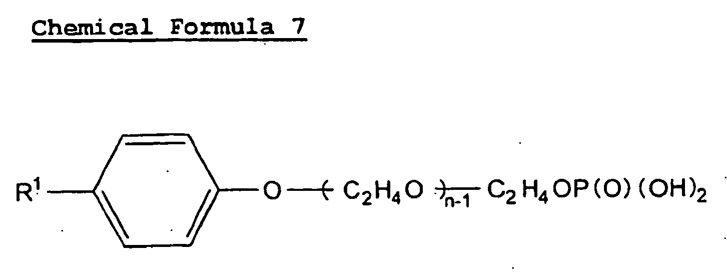 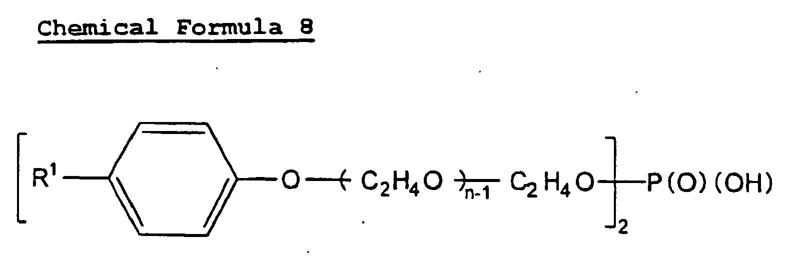 (Note that R¹ in the chemical formulas 7 and 8 is an alkyl group having 7 to 18 carbon atoms, and having 8 to 9 carbon atoms in many cases, and "n" is an average number of moles added which is 1 to 18, and 2 to 8 in many cases) 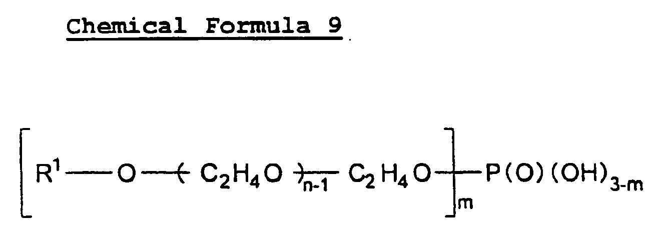 (In the chemical formula 9, R¹ is an alkyl group having 7 to 18 carbon atoms, and having 8 to 9 carbon atoms in many cases, "n" is an average number of moles added which is 1 to 18, and 2 to 8 in many cases, and "m" is an integer of 1 to 3) 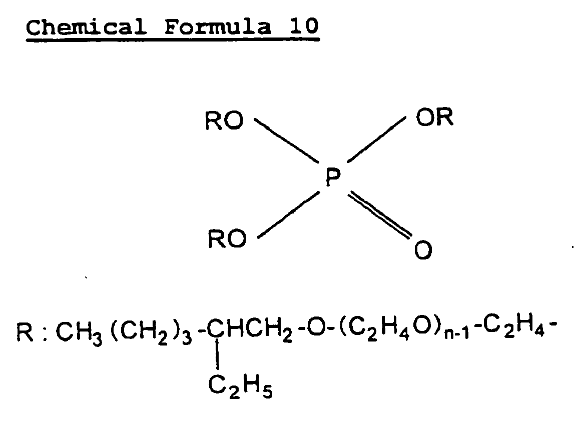 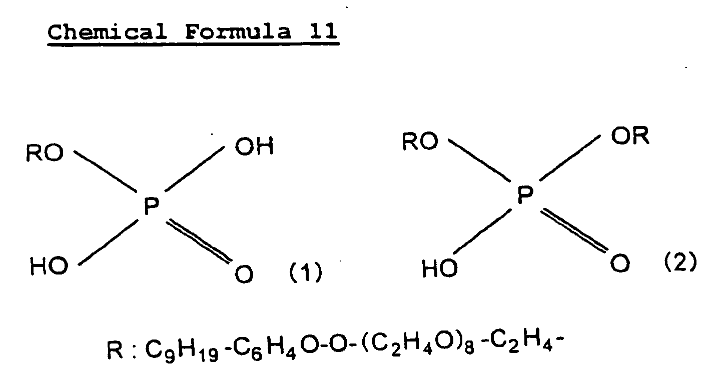 Sodium dihydrogen phosphate, potassium dihydrogen phosphate and ammonium dihydrogen phosphate; sodium hydrogenphosphate, potassium hydrogenphosphate and ammonium hydrogenphosphate; sodium phosphate, potassium phosphate and ammonium phosphate; sodium hexametaphosphate and pentametaphosphate; sodium tripolyphosphate, potassium pyrophosphate, sodium pyrophosphate, and potassium tripolyphosphate;
      potassium pyrophosphate 2-ethylhexylester and sodium pyrophosphate 2-ethylhexylester; monoalkyl phosphate disalts; and triphenyl phosphine.

   Among these phosphorous-containing compounds (c), phosphoric ester based surfactants (normally, used as an anticorrosive agent), phosphoric compounds and alkali metal salt of phosphoric compounds are preferable, and alkali metal dihydrogenphosphate is more preferable.
   A method of supplying a phosphorous-containing compound is, when using a solid or powder compound, preferably dissolving in polar solvents such as water or dimethylformamide (DMF) for supplying in terms of operationality and supply accuracy, and dissolving in water for supplying is particularly preferable in terms of solubility.
   The use ratio of at least one of the compounds (a) and (b) to the compound (c) is normally 1:10 to 100:1, preferably 1:5 to 80:1, more preferably 1:2 to 70:1 in weight ratios.
   A total amount of the compounds (a) and/or (b) and (c) is normally 0.1 to 2000 wtppm based on weight of a monomer mixture including conjugated dienes.
   A method of using a polymerization inhibitor in the present invention is not particularly limited and may be attained by simply bringing conjugated diene or a hydrocarbon mixture including conjugated diene contact the polymerization inhibitor.
(6) An extracting solvent which can be used in the present invention is not particularly limited as far as it can dissolve and extract conjugated dienes and, for example, water, acetone, methylethylketone, dioxane, isoprene cyclic sulfone, acetonitrile, alcohol, glycol, N-methylolamine, N-ethyl succinimide, N-methyl pyrrolidone, N-methyl-2-pyrrolidone, hydroxylethyl pyrrolidone, N-methyl-5-methylpyrrolidone, furfural, 2-heptenone, dimethylformamide (DMF), dimethyl acetoamide, N,N-dimethyl acetone acetic acid amide, morpholine, N-formyl morpholine, N-methyl morpholine-3-one, sulfolane, methylcarbitol, tetrahydrofuran, aniline, N-methyl oxazolidone, N-methyl imidazole, N,N'-dimethyl imidazoline-2-one, 1-oxo-1-methyl phosphorine, methyl cyanoacetate, ethyl acetoacetate, ethylacetate, dimethyl malonate ester, propylene carbonate, methyl carbitol, triethyl phosphate, diethylene glycol monomethyl ether, dimethyl sulfoxide and γ-butyrolactone, etc. may be mentioned, but amide compounds are preferable and dimethylformamide (DMF) is more preferable. These extracting solvent may be used alone or in combination of two or more.

An amount of the extracting solvent used in the present invention is normally 100 to 1000 parts by weight, preferably 200 to 800 parts by weight with respect to 100 parts by weight of a hydrocarbon mixture including conjugated diene. It is preferable to supply the extracting solvent from an extracting solvent supplying stage provided at a position upper than a stage of supplying a hydrocarbon mixture including conjugated diene (petroleum distillates supplying stage) to the extractive distillation column.

In the present invention, to prevent blot of the extractive distillation column (to furthermore prevent generation of a polymer), the extracting solvent is preferably added with heterocyclic aldehyde, an aromatic nitro compound or aromatic aldehyde.

The heterocyclic aldehyde is aldehyde having heterocycle and, for example, furfural, 5-methylfurfural, 5-(hydroxymethyl)furfural; thiophenecarbaldehyde, nicotine aldehyde and pyridoxal, etc. may be mentioned, but furfural is preferable.

An aromatic nitro compound is nitro having an aromatic ring and, for example, nitrobenzene, nitrotoluene, a-nitrotoluene, nitroxylene, nitromesitylene, dinitrobenzene, dinitrotoluene, dinitroxylene, trinitrobenzene and trinitroxylene, etc. may be mentioned, but nitrobenzene is preferable.

An aromatic aldehyde is aldehyde having an aromatic ring and, for example, benzaldehyde, tolualdehyde, cuminaldehyde, phenylacetoaldehyde, cinnamaldehyde, phthal aldehyde, isophthal aldehyde and terephthal aldehyde, etc. may be mentioned, but benzaldehyde is preferable.

Concentration of these heterocyclic aldehyde, an aromatic nitro compound or aromatic aldehyde is normally 0.01 to 10 wt%, preferably 0.05 to 5 wt% in the extracting solvent in the extracting solvent supplying stage.

As explained above, in the case where the extracting solvent is added with heterocyclic aldehyde, an aromatic nitro compound or aromatic aldehyde, to furthermore prevent generation of a polymer, the extracting solvent preferably includes tar of polycondensate of heterocyclic aldehyde or aromatic aldehyde, etc. Concentration of the polycondensate is normally 0.5 to 10 wt%, preferably 1 to 5 wt% in the extracting solvent on the extracting solvent supply stage. When the concentration of the polycondensate becomes too high, extraction efficiency tends to decline, while when it becomes too low, a large amount of heterocyclic aldehyde or aromatic aldehyde in dimethylformamide is consumed which is wasteful.

### Operation and Effect of the Invention

In a method of separating and purifying conjugated dienes a separating and purifying apparatus is used in the present invention, wherein a specific polymerization inhibitor is used, moreover, concentration of the polymerization inhibitor in an extracting solvent to be recovered after extractive distillation for circulation use is measured, and an amount of the polymerization inhibitor to be supplied to an extractive distillation column is changed based thereon. Therefore, it is possible to supply a polymerization inhibitor in just proportion required for suppressing generation of rubbery substances inside the apparatus, and generation of rubbery substances can be efficiently and stably suppressed. As a result, costs of separating and purifying conjugated dienes can be reduced comparing with that in the conventional techniques, moreover, concentration of the polymerization inhibitor in purified conjugated dienes can be suppressed low.

### BRIEF DESCRIPTION OF DRAWINGS

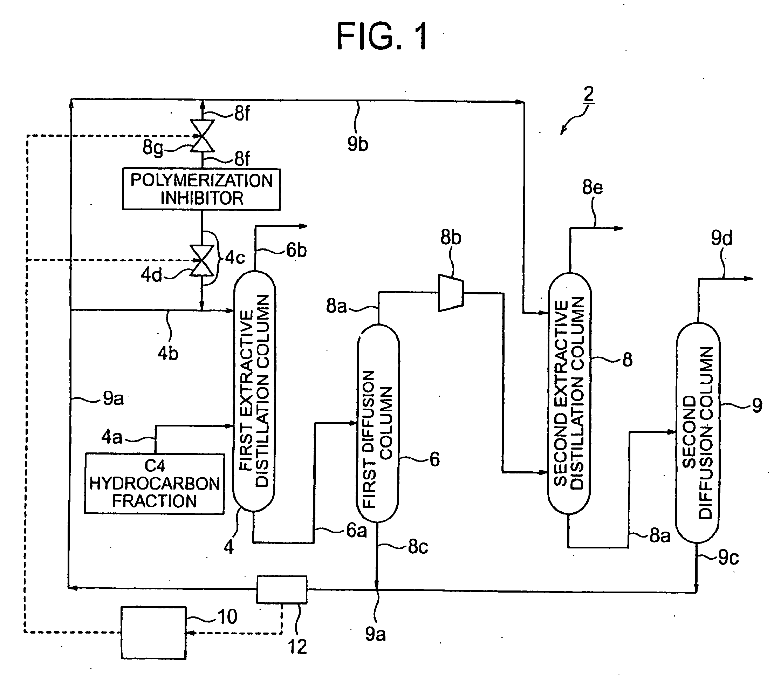

Figure 1 is a block diagram showing an example of a separation and purification system using a separation and purification apparatus.

Below, illustrative embodiments will be explained based on the drawing

In the present embodiment, as an example of an apparatus for separating and purifying conjugated dienes, an explanation will be made by taking an example of a separation and purification apparatus used for separating a C4 hydrocarbon compound from C4 hydrocarbon fraction and purifying butadiene.

### Separation and Purification System 2

As shown in FIG. 1, a separation and purification system 2 according to the present embodiment comprises a first extractive distillation column 4, a first diffusion column 6, a second extractive distillation column 8 and a second diffusion column 9.

The first extractive distillation column 4 is a column for performing extractive distillation of a first stage on a C4 hydrocarbon fraction and taking out a first distillation portion and a first bottom product, which is connected to the later explained first diffusion column 6 by a line 6a. Near an upper stage of the first extractive distillation column 4 is connected a line 4b for supplying a polymerization inhibitor through a line 4c. A supply amount adjustment valve 4d for adjusting a supply amount of the polymerization inhibitor is provided on the line 4c, and a supply amount of the polymerization inhibitor can be adjusted based on an output signal sent from a control device 10.

The first extractive distillation column 4 generally comprises a column body, a condenser for cooling vapor exhausted from top of the column body and condensing to a liquid, a reflux drum for storing the distillation portion condensed to a liquid by the condenser, a reflux line for re-supply a part of the distillation portion stored in the reflux drum to near the top of the column body, and a re-boiler arranged at the bottom of the column body, but they are omitted in the drawing in the present embodiment. A form of the distillation column may be, for example, any of a plate column wherein the inside of the column body is divided by horizontal stage trays, so that contact of the liquid and steam is made gradually, or a packed column filled with filler for efficient material movement between different phases, etc. When using a plate column, the number of stage trays is normally 100 to 300 stages, preferably 150 to 200 stages. Accuracy of distillation (ability of separating those having close boiling points) becomes higher as the number of stage trays becomes larger; however, when the number is too large, the cost is increased, so that it is selected in consideration of balance of the ability and the cost. Note that the reference number "6b" in FIG. 1 is a line of taking out the first distillation portion from the reflux drum (not shown).

The first diffusion column 6 is a column for distilling a first bottom product supplied through the line 6a and taking out a second distillation portion and a second bottom product, and connected to the later explained second extractive distillation column 8 by a line 8a. Note that in the present embodiment, a compressor 8b is provided on the line 8a. Near the bottom of the first diffusion column 6, a line 8c capable of recovering the second bottom product is connected, and the line 8c is connected to lines 9a and 9b capable of circulating. The line 9a is provided with a liquid chromatograph analyzing device (measurement device) 12 for measuring concentration of a polymerization inhibitor included in the second bottom product and the later explained fourth bottom product flowing there, and the measured concentration of the polymerization inhibitor is sent as a predetermined output signal to the control device (control means) 10.

The first diffusion column 6 generally comprises a column body, a condenser for cooling vapor exhausted from top of the column body and condensing to a liquid, a reflux drum for storing the distillation portion condensed to a liquid by the condenser, a reflux line for re-supplying a part of the distillation portion stored in the reflux drum to near the top of the column body, and a re-boiler arranged at the bottom of the column body, but they are omitted in the drawing in the present embodiment in the same way as in the case of first extractive distillation column 4. A form of the first diffusion column 6 may be a plate column or a packed column.

The second extractive distillation column 8 is a column for performing extractive distillation of a second stage on the second distillation portion supplied through the line 8a and taking out a third distillation portion and a third bottom product, and connected to the later explained second diffusion column 9 by a line 8d. Near the upper stage of the second extractive distillation column 8, a line 9b for supplying polymerization inhibitor through a line 8f is connected. The line 8f is provided with a supply amount adjustment valve 8g for adjusting a supply amount of the polymerization inhibitor, and the supply amount of the polymerization inhibitor can be adjusted based on an output signal sent from the control device 10. In the present embodiment, the second extractive distillation column 8 is applied the same configuration as that in the first extractive distillation column 4 explained above, but it is not particularly limited in the present invention. Note that the reference number "8e" in FIG. 1 is a line for taking out a third distillation portion from the reflux drum (not shown).

The second diffusion column 9 is a column for distilling the third bottom product supplied through the line 8d and taking out a fourth distillation portion and a fourth bottom product. Near the bottom of the second diffusion column 9, a line 9c capable of recovering the fourth bottom product is connected, and the line 9c is connected to lines 9a and 9b capable of circulating. The second diffusion column 9 is applied the same configuration as that of the first diffusion column 6 explained above in the present embodiment, but it is not particularly limited in the present invention. Note that the reference number "9d" in FIG. 1 is a line for taking out the fourth distillation portion from the reflux drum (not shown).

### Effects

A separation and purification system 2 according to the present embodiment exhibits effects below.

The first extractive distillation column 4 is supplied with a C4 hydrocarbon portion through a line 4a. A position of supplying the C4 hydrocarbon fraction to the first extractive distillation column 4 is not particularly limited, but normally at an approximate middle stage. Along with the supply of the C4 hydrocarbon fraction, an extracting solvent is supplied from the line 4b, where a polymerization inhibitor is supplied through the line 4c, the polymerization inhibitor is mixed with the extracting solvent in the middle of the line 4b, then, supplied to the first extractive distillation column 4. Along with the supply of these, extractive distillation of the first stage is performed by heating by the re-boiler (not shown) arranged at the bottom of the first extractive distillation column 4. A pressure inside the first extractive distillation column 4 is not particularly limited and is normally 0.1 to 1.0 MPa (1 to 10 atms), and a temperature at the bottom is preferably 100 to 160°C. From near the top of the distillation column 4, a distillation portion including much butane and butene is taken out, the distillation portion is condensed in the condenser (not shown), a part thereof is refluxed to be returned to the top of the column, and the remaining portion is taken out as a first distillation portion through the line 6b. On the other hand, from the bottom of the evaporation column 4, butadiene fraction including much high acetylene and allene based hydrocarbon is taken out as the first bottom product and supplied to the first diffusion column 6 through the line 6a.

A position of supplying the first bottom product to the first diffusion column 6 is not particularly limited, but normally at an approximate middle stage. Along with the supply of the first bottom product, distillation is performed by heating by a re-boiler (not shown) arranged at the bottom of the first diffusion column 6. A pressure in the first diffusion column 6 is normally 0.1 to 0.2 MPa (1 to 2 atms), and a temperature at the bottom is a boiling point under the pressure. A mixed solvent including a polymerization inhibitor and an extraction solvent (hereinafter, also simply referred to as "a mixed solvent") are taken out as a second bottom product from the bottom of the diffusion column 6, and the second bottom product is circulated to the first extractive distillation column 4 via the lines 8c, 9a and 4b or to the second extractive distillation column 8 via the lines 8c, 9a and 9b to be reused. On the other hand, a distillation portion including a large amount of butadiene, high acetylene and allene based hydrocarbon is taken out from the top of the diffusion column 6, the distillation portion is condensed in the condenser (not shown), a part thereof is refluxed to return to the top of the column and the remaining portion is taken out as the second distillation portion and supplied to the second extractive distillation column 8 through the line 8a.

A position of supplying the second distillation portion to the second extractive distillation column 8 is not particularly limited but is normally near a lower stage. Along with the supply of the second distillation portion, a polymerization inhibitor is supplied to the line 9b through the line 8f, the polymerization inhibitor is mixed with the mixed solvent in the middle of the line 9b to be supplied to the second extractive distillation column 8. Along with the supply, extractive distillation of the second stage is performed by heating the re-boiler (not shown) arranged at the bottom of the second extractive distillation column 8. A pressure in the second extractive distillation column 8 is not particularly limited, but normally is 0.1 to 1.0 MPa (1 to 10 atms), and a temperature at the bottom is preferably 100 to 160°C. A distillation portion including much butadiene is taken out from the top of the distillation column 8, the distillation portion is condensed by the condenser (not shown), a part thereof is refluxed to return to the top of the column and the remaining portion is taken out as a third distillation portion through the line 8e. The third distillation portion taken out from the line 8e is then supplied to a distillation column (not shown) to be subjected to further distillation processing and taken out in a form wherein butadiene is highly condensed. Finally, it is supplied as high purity butadiene to a material of polybutadiene, etc. On the other hand, a distillation portion including much mixed solvent is taken out as a third bottom product and supplied to the second diffusion column 9 through the line 8d. Note that a predetermined amount of polymerization inhibitor, such as diethylhydroxylamine (DEHA), may be added from near the top of the second extractive distillation column 8 through a not shown line. Consequently, generation of a pop corn polymer inside the second extractive distillation column 8 can be effectively prevented.

A position of supplying the third bottom product to the second diffusion column 9 is not particularly limited, but normally at an approximate middle stage. Along with the supply of the third bottom product, distillation is performed by heating the re-boiler (not shown) arranged at the bottom of the second diffusion column 9. A pressure in the second diffusion column 9 is normally 1 to 2 atms, and a temperature at the bottom is a boiling point under the pressure. After a predetermined time, a distillation portion including much waste is taken out from the top of the diffusion column 9 as a fourth distillation portion. On the other hand, from the bottom of the diffusion column 9, in the same way as in the first diffusion column 6 explained above, a mixed solvent including polymerization inhibitor and an extracting solvent is taken out as a fourth bottom product, the fourth bottom product is mixed with the above second bottom product and refluxed to return to the first extractive distillation column 4 through the lines 9c, 9a and 4b or to the second extractive distillation column 8 through the lines 9c, 9a and 9b to be reused.

In the present embodiment, the line 9a is provided with a liquid chromatograph analyzing device (measurement device) 12 for measuring concentration of a polymerization inhibitor included in the mixed solvent (the second bottom product and the fourth bottom product) flowing in the line 9a. The measured concentration of the polymerization inhibitor is sent as an output signal (a concentration value) to the control device 10, where the concentration value is compared with a reference range (concentration in a predetermined range). The concentration of the reference range is preferably 0.1 to 20 ppm, more preferably 0.5 to 10 ppm. As a result, when the actually measured concentration value is judged to be too high, the control device 10 sends to the valves 4d and 8g a signal to decrease a supply amount of the polymerization inhibitor to the first extractive distillation column 4 and the second extractive distillation column 8. Inversely, when the concentration of the polymerization inhibitor included in the mixed solvent flowing in the line 9a is judged to be too low, the control device 10 sends to the valves 4d and 8g a signal to increase the supply amount of the polymerization inhibitor to the first extractive distillation column 4 and the second extractive distillation column 8. In the present embodiment, as explained above, on-off control is made to adjust the concentration of the polymerization inhibitor included in the mixed solvent flowing in the line 9a to be in a reference range, but it may be controlled by other control algorithm, for example, proportional control, proportional integral control, proportional integral differentiation control, fuzzy control and adaptive control, etc. Note that the analyzing device (measurement device) 12 is not necessarily on the line, and control may be performed by taking out a small amount of the mixed solvent, measuring the sample while offline, and inputting the measurement result to the control device 10.

According to the separation and purification system 2 according to the present embodiment, it is possible to supply a polymerization inhibitor in just proportion required for suppressing generation of rubbery substances, and generation of rubbery substances can be efficiently and stably suppressed. Also, even when a supply amount of a C4 fraction increases or decreases, an amount of the polymerization inhibitor can be controlled in accordance thereto, so that stable operation becomes possible despite of changes of operation conditions of the system. As a result, by performing further distillation processing by using a distillation portion including much butadiene separated through the line 9d, high purity butadiene can be separated and purified at a lower cost comparing with the conventional cases.

Also, in the present embodiment, by measuring concentration of the polymerization inhibitor included in a mixed solvent flowing in the line 9a by using a liquid chromatograph analyzing device 12, measurement of the concentration of the polymerization inhibitor can be made easily and efficiently.

Note that in the present embodiment, since an output signal is continuously sent from the analyzing device 12 to the control device 10, the above operation can also be continuously performed in the control device 10. However, to reduce costs and troubles of measurement, the measurement interval is preferably set to several hours to several days, preferably 8 hours to 10 days, and digital controlling is preferable.

Embodiments of the present invention were explained above. The present invention may be naturally carried out in various embodiments within the scope of the present claims.

For example, in the above embodiment, a C4 hydrocarbon compound is separated from C4 hydrocarbon fraction to purify butadiene, but a C5 hydrocarbon compound may be separated from C5 hydrocarbon fraction to purify isoprene.

## Claims

1. A method of separating and purifying conjugated dienes, including:
a step of obtaining a first bottom product by taking out a hydrocarbon mixture including conjugated diene under an environment in the presence of a polymerization inhibitor including at least one of nitrite and
di-lower alkylhydroxylamine defined by chemical formula 1: 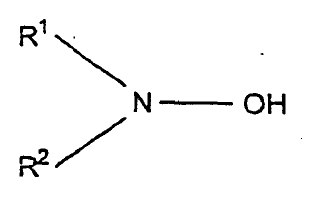 wherein R¹ and R² are linear, branched or cyclic alkyl groups having 1 to 10 carbon atoms, respectively,
in an extractive distillation column;
a step of obtaining a second bottom product by distilling said first bottom product in a diffusion column;
a step of recovering said second bottom product and supplying to said extractive distillation column;
a step of measuring concentration of a polymerization inhibitor included in said second bottom product; and
a control step of controlling the concentration of the polymerization inhibitor included in said second bottom product by changing a supply amount of the polymerization inhibitor to said extractive distillation column in accordance with said measured concentration of the polymerization inhibitor.

2. The method of separating and purifying conjugated dienes as set forth in claim 1, wherein nitrite is sodium nitrite or potassium nitrite.

3. The method of separating and purifying conjugated dienes as set forth in claim 1, wherein di-lower alkylhydroxylamine is diethylhydroxylamine.

4. The method of separating and purifying conjugated dienes as set forth in claim 1, wherein a polymerization inhibitor further includes a phosphorous-containing compound selected from phosphoric acid, phosphonic acid, phosphinic acid, diphosphonic acid, diphosphoric acid, tripolyphosphoric acid, metaphosphoric acid, alkyl dihydrogenphosphate as defined by chemical formula 2, dialkyl hydrogenphosphate as defined by chemical formula 3, and trialkyl phosphate as defined by chemical formula 4, 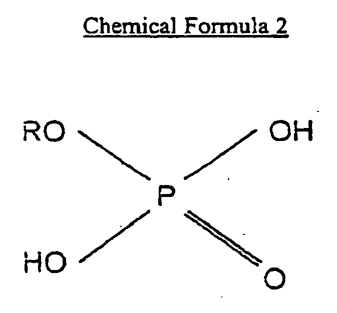 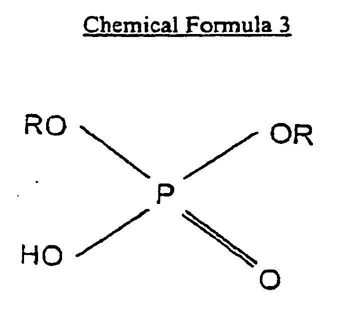 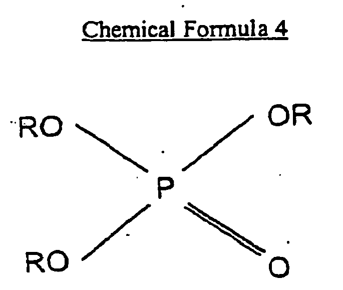 wherein R in the chemical formulas 2 to 4 includes hydrocarbon groups, or hydrophobic groups,
triphenylphosphate as defined by chemical formula 5, 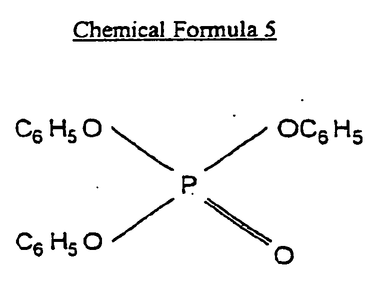 tris(nonylphenyl)phosphite defined by chemical formula 6, 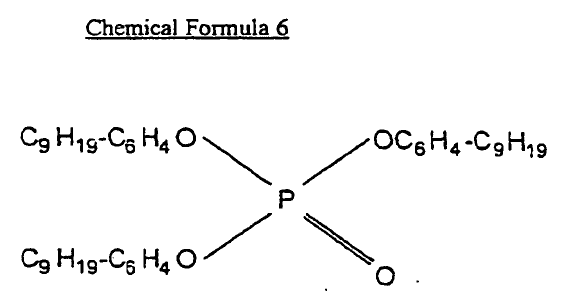 dimethyl phosphonate, diethyl phosphonate, triisopropyl phosphonate, triphenyl phosphonate, metaphosphate, phosphoric ester based surfactants as defined by chemical formulas 7, 8, 9 and 10, 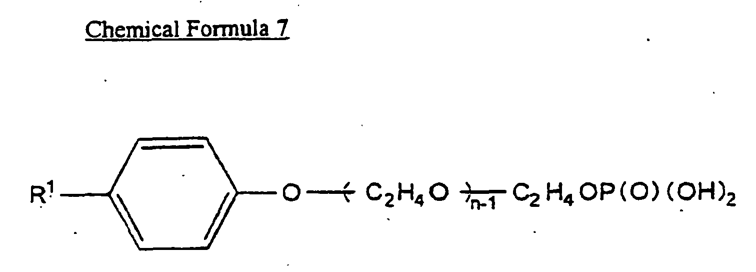 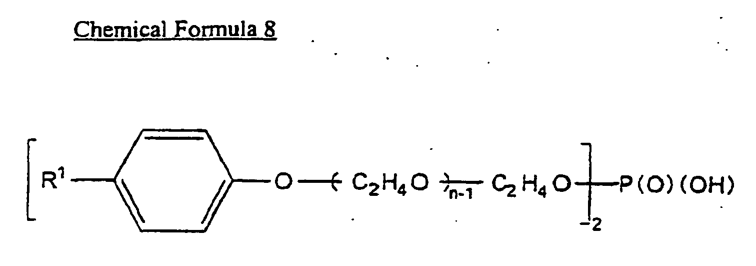 wherein R¹ in the chemical formulas 7 and 8 is an alkyl group having 7 to 18 carbon atoms, and "n" is an average number of moles added which is 1 to 18, 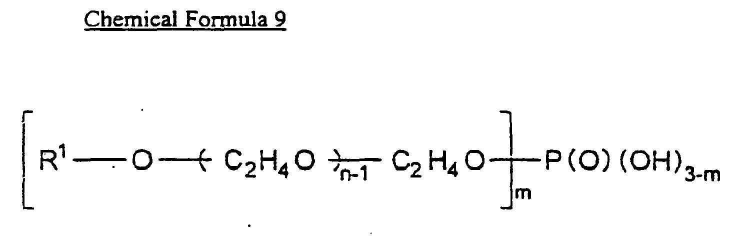 wherein R¹ in the chemical formula 9, is an alkyl group having 7 to 18 carbon atoms, "n" is an average number of moles added which is 1 to 18, and "m" is an integer of 1 to 3, 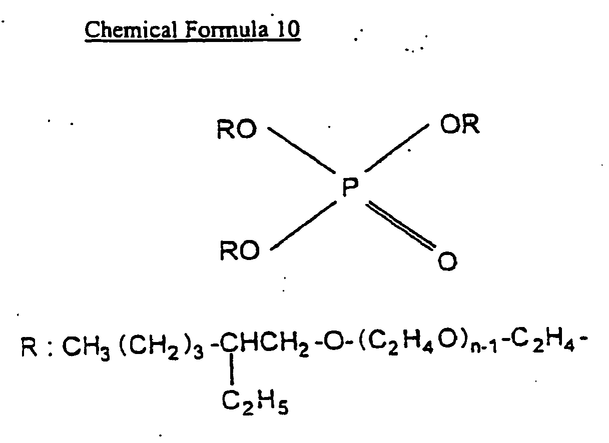 and a compound mixture expressed by the chemical formula 11 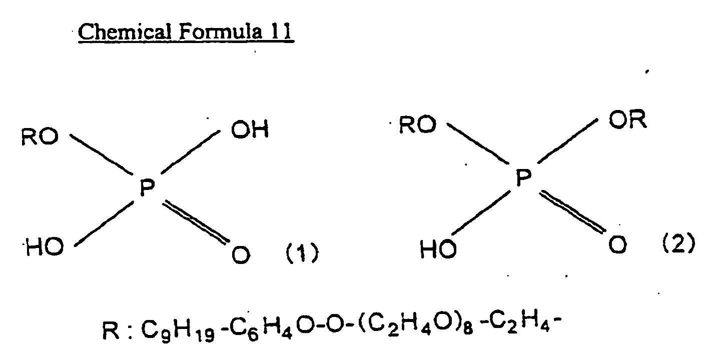 and other esterification products of phosphoric compounds, sodium dihydrogen phosphate, potassium dihydrogen phosphate, ammonium dihydrogen phosphate, sodium hydrogenphosphate, potassium hydrogenphosphate, ammonium hydrogenphosphate; sodium phosphate, potassium phosphate, ammonium phosphate, sodium hexametaphosphate, sodium pentametaphosphate, sodium tripolyphosphate, potassium pyrophosphate, sodium pyrophosphate, potassium tripolyphosphate, potassium pyrophosphate 2-ethylhexylester, sodium pyrophosphate 2-ethylhexylester, monoalkyl phosphate disalts, and triphenyl phosphine.

5. The method of separating and purifying conjugated dienes as set forth in claim 4, wherein a phosphorous-containing compound is an alkali metal dihydrogenphosphate.

6. The method of separating and purifying conjugated dienes as set forth in claim 4, wherein total weight of nitrite, di-lower alkylhydroxylamine as defined in claim 1 and a phosphorous-containing compound as defined in claim 4 is 0.1 to 2000 wtppm based on weight of a monomer mixture including conjugated dienes.

7. The method of separating and purifying conjugated dienes as set forth in claim 4, wherein a use ratio of at least one of nitrite and di-lower alkylhydroxylamine as defined in claim 1 to a phosphorous-containing compound as defined in claim 4 is 1:2 to 70:1 in weight ratio.

8. The method of separating and purifying conjugated dienes as set forth in claim 1, wherein an extracting solvent used in extractive distillation is dimethylformamide.

9. The method of separating and purifying conjugated dienes as set forth in claim 1, wherein a use amount of an extracting solvent used in extractive distillation is 200 to 800 parts by weight with respect to 100 parts by weight of a hydrocarbon mixture including conjugated diene.

10. The method of separating and purifying conjugated dienes as set forth in claim 1, wherein an extracting solvent used in extractive distillation includes heterocyclic aldehyde, an aromatic nitro compound or aromatic aldehyde.

11. The method of separating and purifying conjugated dienes as set forth in claim 1, wherein a hydrocarbon mixture including conjugated diene is a hydrocarbon mixture of C4 or more.

12. The method of separating and purifying conjugated dienes as set forth in claim 1, wherein a hydrocarbon mixture including conjugated diene is C4 hydrocarbon fraction including butadiene or C5 hydrocarbon fraction including isoprene.

13. Use of an apparatus for separating and purifying conjugated dienes, wherein the apparatus comprises:
an extractive distillation column for obtaining a first bottom product by taking out a hydrocarbon mixture including conjugated diene under an environment in the presence of a polymerization inhibitor including at least one of nitrite and di-lower alkylhydroxylamine as defined in claim 1;
a diffusion column for obtaining a second bottom product by distilling said first bottom product;
a first supply system for recovering said second bottom product and supplying to said extractive distillation column;
a measurement means for measuring concentration of a polymerization inhibitor included in said second bottom product;
a second supply system for supplying said polymerization inhibitor newly to said extractive distillation column; and
a control means for controlling concentration of the polymerization inhibitor included in said second bottom product by changing a supply amount of the polymerization inhibitor to said extractive distillation column in accordance with said measured concentration of the polymerization inhibitor.

## Patentansprüche

1. Ein Verfahren zum Trennen und Reinigen von konjugierten Dienen, enthaltend:
einen Schritt des Erhaltens eines ersten Sumpfprodukts durch Entnehmen eines Kohlenwasserstoffgemisches, das konjugiertes Dien enthält, in einer Umgebung in Gegenwart eines Polymerisationsinhibitors, der mindestens eines aus Nitrit und di-Niederalkylhydroxylamin enthält, wobei das di-Niederalkylhydroxylamin durch die chemische Formel 1 definiert ist: 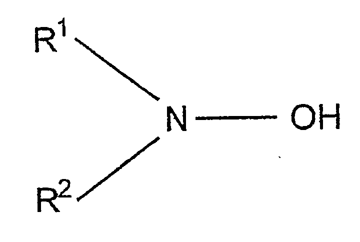 wobei R¹ und R² jeweils lineare, verzweigte oder cyclische Alkylreste mit 1 bis 10 Kohlenstoffatomen sind,
in einer extraktiven Destillationssäule;
einen Schritt des Erhaltens eines zweiten Sumpfprodukts durch Destillieren des ersten Sumpfprodukts in einer Diffusionssäule;
einen Schritt des Gewinnens des zweiten Sumpfprodukts und Zuführens zur extraktiven Destillationssäule;
einen Schritt des Messens der Konzentration eines Polymerisationsinhibitors, der im zweiten Sumpfprodukt enthalten ist; und
einen Regelschritt des Regulierens der Konzentration des Polymerisationsinhibitors, der im zweiten Sumpfprodukt enthalten ist, durch Verändern einer Zufuhrmenge des Polymerisationsinhibitors zur extraktiven Destillationssäule gemäß der gemessenen Konzentration des Polymerisationsinhibitors.

2. Das Verfahren zum Trennen und Reinigen von konjugierten Dienen wie in Anspruch 1 beansprucht, wobei Nitrit Natriumnitrit oder Kaliumnitrit ist.

3. Das Verfahren zum Trennen und Reinigen von konjugierten Dienen wie in Anspruch 1 beansprucht, wobei das di-Niederalkylhydroxylamin Diethylhydroxylamin ist.

4. Das Verfahren zum Trennen und Reinigen von konjugierten Dienen wie in Anspruch 1 beansprucht, wobei ein Polymerisationsinhibitor ferner eine phosphorhaltige Verbindung, ausgewählt aus Phosphorsäure, Phosphonsäure, Phosphinsäure, Diphosphonsäure, Diphosphorsäure, Tripolyphosphorsäure, Metaphosphorsäure, Alkyldihydrogenphosphat, wie durch die chemische Formel 2 definiert, Dialkylhydrogenphosphat, wie durch die chemische Formel 3 definiert, und Trialkylphosphat, wie durch die chemische Formel 4 definiert, 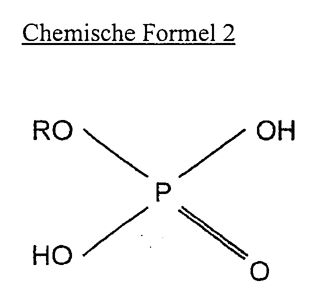 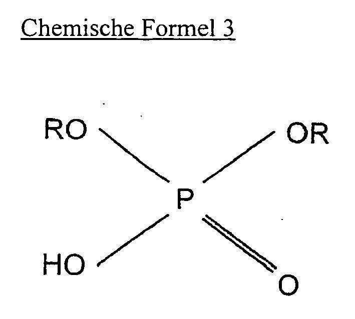 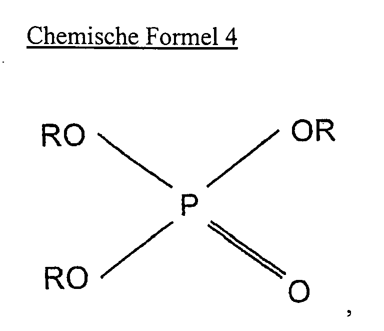 wobei R in den chemischen Formeln 2 bis 4 Kohlenwasserstoffreste oder hydrophobe Reste enthält,
Triphenylphosphat, wie durch die chemische Formel 5 definiert, 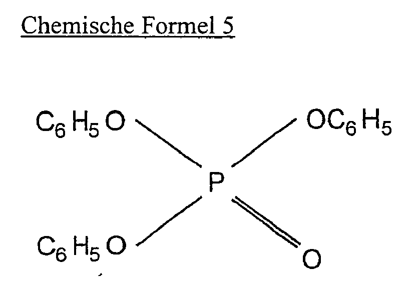 Tris(nonylphenyl)phosphit, wie durch die chemische Formel 6 definiert, 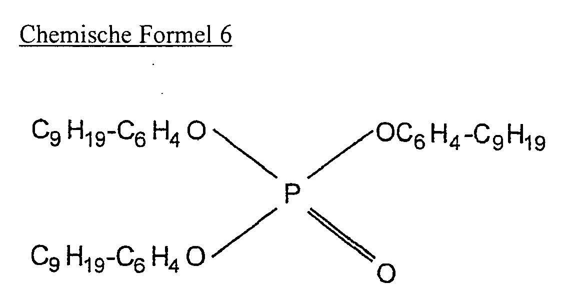 Dimethylphosphonat, Diethylphosphonat, Triisopropylphosphonat, Triphenylphosphonat, Metaphosphat, grenzflächenaktive Mittel auf der Basis von Phosphorsäureester, wie durch die chemischen Formeln 7, 8, 9 und 10 definiert, 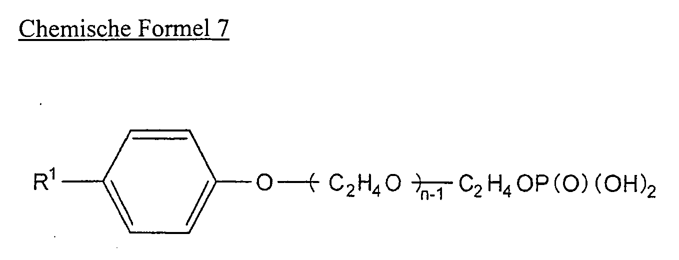 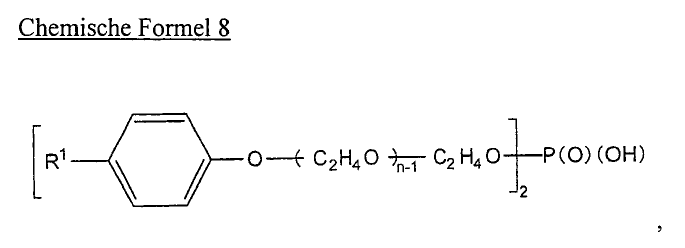 wobei R¹ in den chemischen Formeln 7 und 8 ein Alkylrest mit 7 bis 18 Kohlenstoffatomen ist und "n" eine durchschnittliche Anzahl der addierten Mole ist, die 1 bis 18 beträgt, 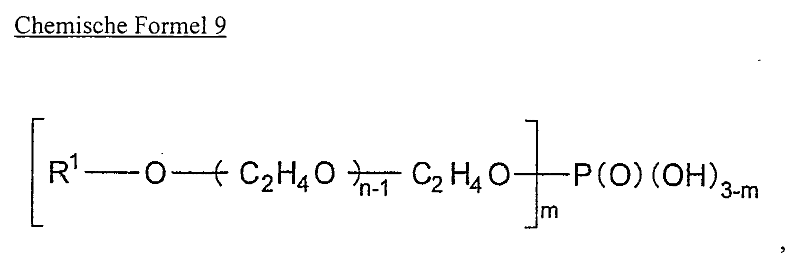 wobei R¹ in der chemischen Formel 9 ein Alkylrest mit 7 bis 18 Kohlenstoffatomen ist, "n" eine durchschnittliche Anzahl der addierten Mole ist, die 1 bis 18 beträgt, und "m" eine ganze Zahl von 1 bis 3 ist, 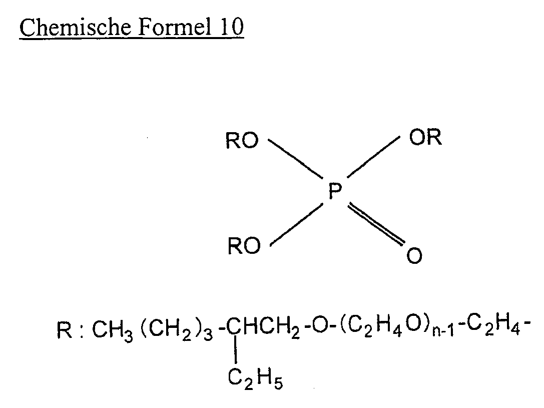 und ein Verbindungsgemisch, dargestellt durch die chemische Formel 11 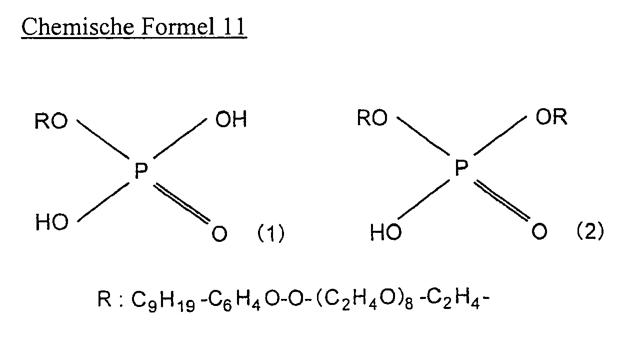 und weitere Veresterungsprodukte von Phosphorsäureverbindungen, Natriumdihydrogenphosphat, Kaliumdihydrogenphosphat, Ammoniumdihydrogenphosphat, Natriumhydrogenphosphat, Kaliumhydrogenphosphat, Ammoniumhydrogenphosphat; Natriumphosphat, Kaliumphosphat, Ammoniumphosphat, Natriumhexametaphosphat, Natriumpentametaphosphat, Natriumtripolyphosphat, Kaliumpyrophosphat, Natriumpyrophosphat, Kaliumtripolyphosphat, Kaliumpyrophosphat-2-ethylhexylester, Natriumpyrophosphat-2-ethylhexylester, Monoalkylphosphatdisalze und Triphenylphosphin enthält.

5. Das Verfahren zum Trennen und Reinigen von konjugierten Dienen, wie in Anspruch 4 beansprucht, wobei eine phosphorhaltige Verbindung ein Alkalimetalldihydrogenphosphat ist.

6. Das Verfahren zum Trennen und Reinigen von konjugierten Dienen, wie in Anspruch 4 beansprucht, wobei das Gesamtgewicht des Nitrits, di-Niederalkylhydroxylamins, wie in Anspruch 1 definiert, und einer phosphorhaltigen Verbindung, wie in Anspruch 4 definiert, 0,1 bis 2000 Gew.-ppm beträgt, bezogen auf das Gewicht eines Monomergemisches, das konjugierte Diene enthält.

7. Das Verfahren zum Trennen und Reinigen von konjugierten Dienen wie in Anspruch 4 beansprucht, wobei ein Einsatzverhältnis von mindestens einem aus Nitrit und di-Niederalkylhydroxylamin, wie in Anspruch 1 definiert, zu einer phosphorhaltigen Verbindung, wie in Anspruch 4 definiert, 1:2 bis 70:1 als Gewichtsverhältnis beträgt.

8. Das Verfahren zum Trennen und Reinigen von konjugierten Dienen wie in Anspruch 1 beansprucht, wobei ein Lösungsmittel zur Extraktion, das bei der extraktiven Destillation verwendet wird, Dimethylformamid ist.

9. Das Verfahren zum Trennen und Reinigen von konjugierten Dienen wie in Anspruch 1 beansprucht, wobei eine Einsatzmenge eines Lösungsmittels zur Extraktion, das bei der extraktiven Destillation verwendet wird, 200 bis 800 Gewichtsteile beträgt, bezogen auf 100 Gewichtsteile eines Kohlenwasserstoffgemisches, das konjugiertes Dien enthält.

10. Das Verfahren zum Trennen und Reinigen von konjugierten Dienen wie in Anspruch 1 beansprucht, wobei ein Lösungsmittel zur Extraktion, das bei der extraktiven Destillation verwendet wird, heterocyclisches Aldehyd, eine aromatische Nitroverbindung oder aromatisches Aldehyd enthält.

11. Das Verfahren zum Trennen und Reinigen von konjugierten Dienen wie in Anspruch 1 beansprucht, wobei ein Kohlenwasserstoffgemisch, das konjugiertes Dien enthält, ein Kohlenwasserstoffgemisch mit C4 oder mehr ist.

12. Das Verfahren zum Trennen und Reinigen von konjugierten Dienen wie in Anspruch 1 beansprucht, wobei ein Kohlenwasserstoffgemisch, das konjugiertes Dien enthält, eine C4-Kohlenwasserstofffraktion, die Butadien enthält, oder eine C5-Kohlenwasserstofffraktion, die Isopren enthält, ist.

13. Verwendung einer Vorrichtung zum Trennen und Reinigen von konjugierten Dienen, wobei die Vorrichtung umfasst:
eine extraktive Destillationssäule zum Erhalten eines ersten Sumpfprodukts durch Entnehmen eines Kohlenwasserstoffgemisches, das konjugiertes Dien enthält, in einer Umgebung in Gegenwart eines Polymerisationsinhibitors, der mindestens eines aus Nitrit und di-Niederalkylhydroxylamin enthält, wie in Anspruch 1 definiert;
eine Diffusionssäule zum Erhalten eines zweiten Sumpfprodukts durch Destillieren des ersten Sumpfprodukts;
ein erstes Zufuhrsystem zum Gewinnen des zweiten Sumpfprodukts und Zuführen zur extraktiven Destillationssäule;
eine Messvorrichtung zum Messen der Konzentration eines Polymerisationsinhibitors, der im zweiten Sumpfprodukt enthalten ist;
ein zweites Zufuhrsystem zum frischen Zuführen des Polymerisationsinhibitors zur extraktiven Destillationssäule; und
eine Regelvorrichtung zum Regulieren der Konzentration des Polymerisationsinhibitors, der im zweiten Sumpfprodukt enthalten ist, durch Verändern einer Zufuhrmenge des Polymerisationsinhibitors zur extraktiven Destillationssäule gemäß der gemessenen Konzentration des Polymerisationsinhibitors.

## Revendications

1. Procédé de séparation et de purification de diènes conjugués, incluant :
une étape d'obtention d'un premier produit de fond en prélevant un mélange hydrocarboné incluant un diène conjugué dans un environnement en présence d'un inhibiteur de polymérisation incluant au moins l'un d'un nitrite et d'une di-alkyle inférieur hydroxylamine définie par la formule chimique 1 : 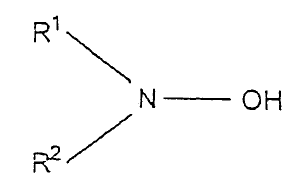 où R¹ et R² sont des groupes alkyle linéaires, ramifiés ou cycliques ayant 1 à 10 atomes de carbone, respectivement,
dans une colonne de distillation extractive ;
une étape d'obtention d'un second produit de fond en distillant ledit premier produit de fond dans une colonne de diffusion ;
une étape de récupération dudit second produit de fond et d'apport à ladite colonne de distillation extractive ;
une étape de mesure de la concentration d'un inhibiteur de polymérisation inclus dans ledit second produit de fond ; et
une étape de commande de la concentration de l'inhibiteur de polymérisation inclus dans ledit second produit de fond en changeant une quantité d'apport de l'inhibiteur de polymérisation à ladite colonne de distillation extractive selon ladite concentration mesurée de l'inhibiteur de polymérisation.

2. Procédé de séparation et de purification de diènes conjugués selon la revendication 1, où le nitrite est le nitrite de sodium ou le nitrite de potassium.

3. Procédé de séparation et de purification de diènes conjugués selon la revendication 1, où la di-alkyle inférieure hydroxylamine est la diéthylhydroxylamine.

4. Procédé de séparation et de purification de diènes conjugués selon la revendication 1, où un inhibiteur de polymérisation inclut en outre un composé contenant du phosphore choisi parmi l'acide phosphorique, l'acide phosphonique, l'acide phosphinique, l'acide diphosphonique, l'acide diphosphorique, l'acide tripolyphosphorique, l'acide métaphosphorique, un dihydrogénophosphate d'alkyle tel que défini par la formule chimique 2, un hydrogénophosphate de dialkyle tel que défini par la formule chimique 3 et un phoshate de trialkyle tel que défini par la formule chimique 4, 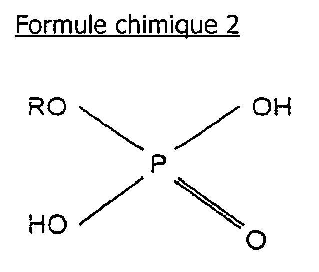 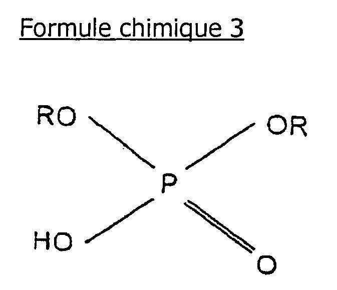 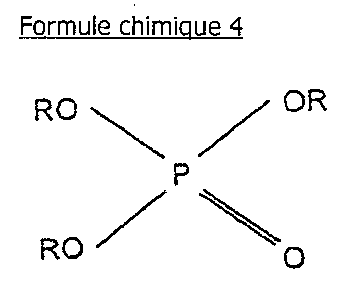 où R dans les formules chimiques 2 à 4 inclut des groupes hydrocarbonés ou des groupes hydrophobes,
un phosphate de triphényle tel que défini par la formule chimique 5 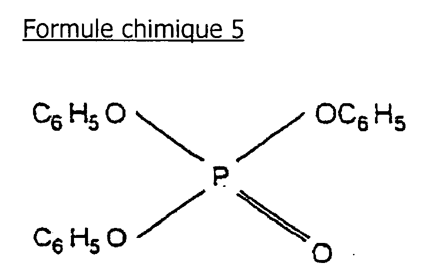 un phosphite de tris(nonylphényl) défini par la formule chimique 6 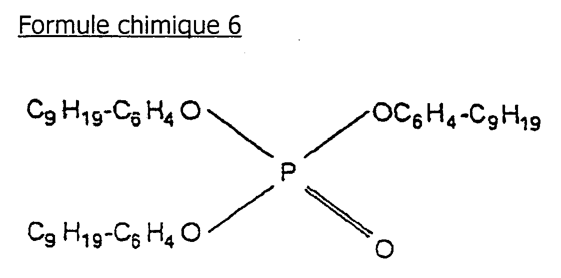 le phosphonate de diméthyle, le phosphonate de diéthyle, le phosphonate de triisopropyle, le phosphonate de triphényle, le métaphosphate, les tensioactifs à base d'esters phosphoriques tels que définis par les formules chimiques 7, 8, 9 et 10 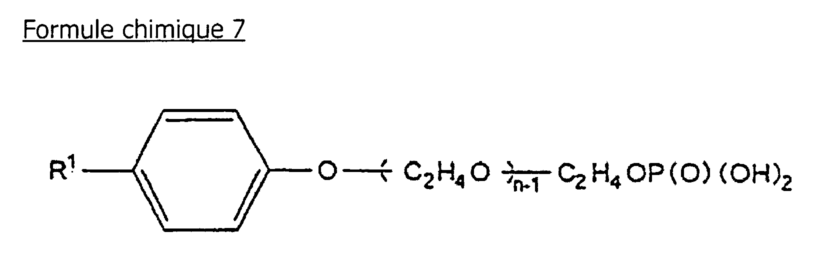 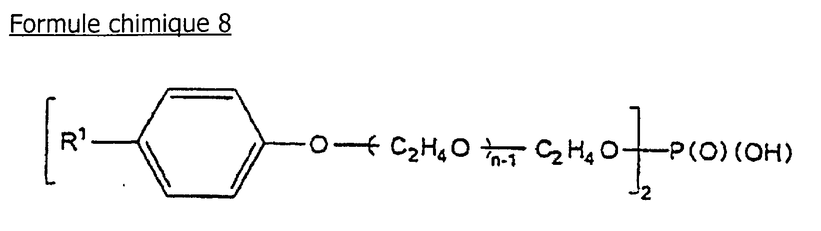 où R¹ dans les formules chimiques 7 et 8 est un groupe alkyle ayant 7 à 18 atomes de carbone, et "n" est un nombre moyen de moles ajoutées qui est 1 à 18, 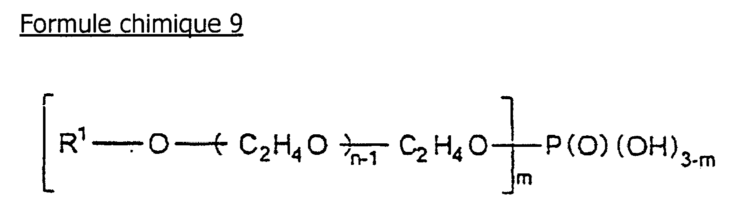 où R¹ dans la formule chimique 9 est un groupe alkyle ayant 7 à 18 atomes de carbone, "n" est un nombre moyen de moles ajoutées qui est 1 à 18, et "m" est un entier de 1 à 3, 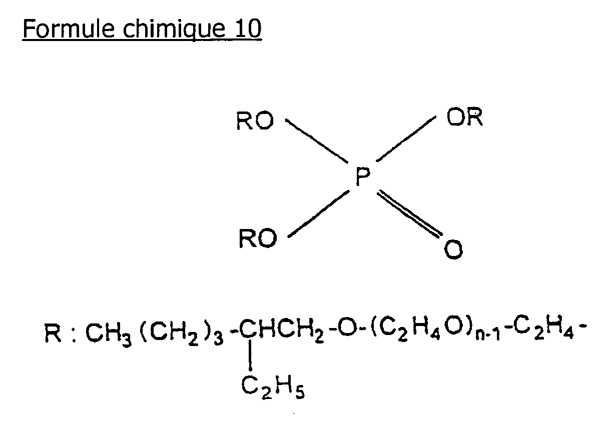 et un mélange de composés exprimé par la formule chimique 11 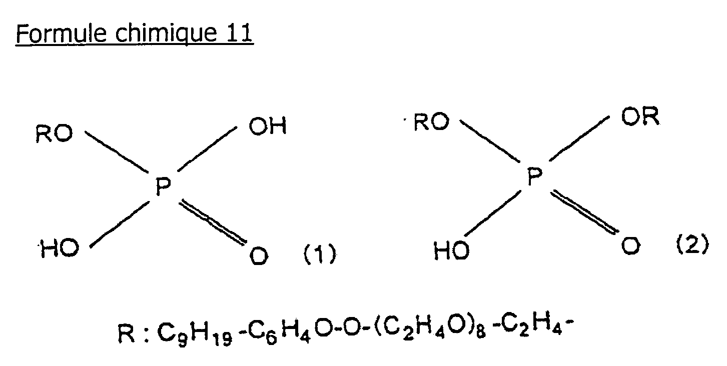 et d'autres produits d'estérification de composés phosphoriques, le dihydrogénophosphate de sodium, le dihydrogénophosphate de potassium, le dihydrogénophosphate d'ammonium, l'hydrogénophosphate de sodium, l'hydrogénophosphate de potassium, l'hydrogénophosphate d'ammonium ; phosphate de sodium, le phosphate de potassium, le phosphate d'ammonium, l'hexamétaphosphate de sodium, le pentamétaphosphate de sodium, le tripolyphosphate de sodium, le pyrophosphate de potassium, le pyrophosphate de sodium, le tripolyphosphate de potassium, le 2-éthylhexylester de pyrophosphate de potassium, le 2-éthylhexylester de pyrophosphate de sodium, les disels de phosphates de monoalkyle et la triphénylphosphine.

5. Procédé de séparation et de purification de diènes conjugués selon la revendication 4, où un composé contenant du phosphore est un dihydrogénophosphate de métal alcalin.

6. Procédé de séparation et de purification de diènes conjugués selon la revendication 4, où le poids total de nitrite, de di-alkyle inférieur hydroxylamine telle que définie dans la revendication 1 et d'un composé contenant du phosphore tel que défini dans la revendication 4 est 0,1 à 2 000 ppm en poids sur la base du poids d'un mélange de monomères incluant des diènes conjugués.

7. Procédé de séparation et de purification de diènes conjugués selon la revendication 4, où un rapport d'utilisation d'au moins l'un d'un nitrite et d'une di-alkyle inférieur hydroxylamine telle que définie dans la revendication 1 à un composé contenant du phosphore tel que défini dans la revendication 4 est 1:2 à 70:1 en rapport en poids.

8. Procédé de séparation et de purification de diènes conjugués selon la revendication 1, où un solvant d'extraction utilisé dans la distillation extractive est le diméthylformamide.

9. Procédé de séparation et de purification de diènes conjugués selon la revendication 1, où une quantité d'utilisation d'un solvant d'extraction utilisé dans la distillation extractive est 200 à 800 parties en poids pour 100 parties en poids d'un mélange hydrocarboné incluant un diène conjugué.

10. Procédé de séparation et de purification de diènes conjugués selon la revendication 1, où un solvant d'extraction utilisé dans la distillation extractive inclut un aldéhyde hétérocyclique, un composé nitro aromatique ou un aldéhyde aromatique.

11. Procédé de séparation et de purification de diènes conjugués selon la revendication 1, où un mélange hydrocarboné incluant un diène conjugué est un mélange hydrocarboné de C4 ou plus.

12. Procédé de séparation et de purification de diènes conjugués selon la revendication 1, où un mélange hydrocarboné incluant un diène conjugué est une fraction hydrocarbonée C4 incluant du butadiène ou une fraction hydrocarbonée C5 incluant de l'isoprène.

13. Utilisation d'un appareil pour séparer et purifier des diènes conjugués, où l'appareil comprend :
une colonne de distillation extractive pour obtenir un premier produit de fond en prélevant un mélange hydrocarboné incluant un diène conjugué dans un environnement en présence d'un inhibiteur de polymérisation incluant au moins l'un d'un nitrite et d'une di-alkyle inférieur hydroxylamine telle que définie dans la revendication 1 ;
une colonne de diffusion pour obtenir un second produit de fond par distillation dudit premier produit de fond ;
un premier système d'apport pour récupérer ledit second produit de fond et apporter à ladite colonne de distillation extractive ;
un moyen de mesure pour mesurer la concentration d'un inhibiteur de polymérisation inclus dans ledit second produit de fond ;
un second système d'apport pour apporter ledit inhibiteur de polymérisation nouvellement à ladite colonne de distillation extractive ; et
un moyen de commande pour commander la concentration de l'inhibiteur de polymérisation inclus dans ledit second produit de fond en changeant
une quantité d'apport de l'inhibiteur de polymérisation à ladite colonne de distillation extractive selon ladite concentration mesurée de l'inhibiteur de polymérisation.
